# EUROPÄISCHE PATENTANMELDUNG

(11) **EP 1 205 442 A1**
(43) Veröffentlichungstag der Anmeldung: **15.05.2002**
(21) Anmeldenummer: 00124408.6
(22) Anmeldetag: 08.11.2000
(51) Int. Cl.: C02F 3/28

(54) **Verfahren zur Trennung von Gas, Wasser und Biomasse und Dreiphasentrennsystem**

(71) Anmelder: VA TECH WABAG Deutschland GmbH & Co. KG, 10707 Berlin (DE)
(72) Erfinder:
(74) Vertreter: VA TECH Patente GmbH & Co

(57) **Zusammenfassung**

Gezeigt wird ein Verfahren zur Trennung von Gas, Wasser und Biomasse in einem Schlammbettreaktor (1) zur anaeroben Reinigung von Abwasser durch Vergärung organischer Abwasserinhaltstoffe mittels einer fluidisierbaren, Agglomerate enthaltenden Biomasse, wobei über dem Schlammbett mittels Dreiphasentrennsystem einerseits Biomasse im System zurückgehalten, Biogas abgeleitet und behandeltes Abwasser separat aus dem Reaktor abgezogen wird und wobei zumindest eine Flüssigkeitswalze erzeugt wird, sowie ein entsprechendes Dreiphasentrennsystem. Die Drehgeschwindigkeit der Flüssigkeitswalze wird bei Bedarf gesteuert. Dadurch wird erreicht, dass bei unterschiedlichen Belastungen eine ausreichende Trennung von Biogas und Agglomeraten erfolgt und somit eine verbesserte Ablaufqualität erzielt wird.

## Beschreibung

Die Erfindung betrifft ein Verfahren zur Trennung von Gas, Wasser und Biomasse in einem Schlammbettreaktor nach dem Oberbegriff von Anspruch 1 sowie ein Dreiphasentrennsystem nach dem Oberbegriff von Anspruch 6.

In der anaeroben Abwasserreinigung werden organische Abwasserinhaltsstoffe durch agglomerierte und fluidisierbare Mikroorganismen gereinigt. Die Agglomerate der Biomasse bilden ― in nicht fluidisiertem Zustand ― ein Schlammbett im Bodenbereich des Reaktors, in welches das zu behandelnde Abwasser eingemischt wird. Über dem Schlammbett wird mit einem Dreiphasentrennsystem einerseits Biomasse im Verfahren zurückgehalten und andererseits behandeltes Abwasser sowie das bei der Vergärung gebildete Biogas separat aus dem Verfahren abgezogen.

In einem anaeroben Reinigungsverfahren werden die organischen Abwasserinhaltsstoffe durch Vergären abgebaut, soweit sie diesem Prozess zugänglich sind. Dabei wird Biogas gebildet, welches in seiner Menge und dem zeitlichen Anfall vorwiegend von der Konzentration und der Fracht der organischen Abwasserinhaltsstoffe abhängt. Im Reaktor liegen drei Phasen nebeneinander vor:
1. Flüssigkeit, nämlich das zu behandelnde Abwasser,
2. Feststoffe, nämlich die als agglomerierter Schlamm vorliegende Biomasse und auch mit dem Zulauf eingetragene Partikel und
3. Gas, nämlich das Abbauprodukt Biogas, welches ein Gemisch hauptsächlich aus Methan und Kohlendioxid ist.

Anaerobe Reinigungsverfahren werden günstigerweise in Schlammbettreaktoren, sogenannten UASB-Reaktoren (Upflow Anaerobic Sludge Blanket), durchgeführt. Das Abwasser wird in einem solchen Reaktor im Bodenbereich des Schlammbettes eingemischt. Ein UASB Reaktor ist beispielsweise aus EP 244 029 B1 oder der EP 808 805 A1 bekannt. Die Erfindung bezieht sich auch auf diesen Reaktortyp.

Die Biomasse liegt in einem Schlammbettreaktor in der Regel in Form eines Gemisches vor, welches sich aus sphärischen oder ellipsoiden Agglomeraten von Mikroorganismen und auch aus flockigem Feinschlamm zusammensetzt, wobei die Durchmesser der Agglomerate rund 1 ― 5 mm betragen. Neben den Mikroorganismen können auch Fällungsprodukte zum Aufbau der Agglomerate beitragen.

Die Dichte der Agglomerate ist größer als die des Wassers, daher bildet sich durch die Sedimentation ein Schlammbett im Bodenbereich des Reaktors aus. Das beim Abbau der organischen Abwasserinhaltsstoffe produzierte Biogas bildet Blasen, die sich im Schlammbett nach oben bewegen. Die Blasen haften zunächst an den Agglomeraten an, bis sie sich durch Scherbeanspruchungen und/oder eine ausreichenden Größe von diesen lösen. Die Aufstiegsgeschwindigkeit der Blasen ist durch ihre Größe bestimmt, die auch durch die Druckverhältnisse im Bodenbereich des Reaktors bestimmt werden. Insgesamt ist die Blasenaufstiegsgeschwindigkeit so hoch, dass die Biomasse fluidisiert wird und sich ein Schwebebett ausbilden kann. Dieses Schwebebett weist einen relativ geringeren Anteil an Biomasse auf als ein Schlammbett und nimmt damit ein größeres Volumen ein als dieses.

Aus der zugegebenen Fracht an organischen Abwasserinhaltsstoffen kann eine bestimmte Menge Biogas erzeugt werden. In Abhängigkeit von der Menge des Biogases wird ein bestimmter Teil des Schlammbettes durch die aufsteigenden Gasblasen fluidisiert und liegt als Schwebebett vor, wobei ein Anteil der Agglomerate zusätzlich durch anhaftende Gasbläschen auftreibt.

Schlammbettreaktoren enthalten Dreiphasentrennsysteme mit denen sich Biogas, behandeltes Abwasser und Biomasse voneinander trennen lassen. Dreiphasentrennsysteme können je nach Gewichtung der unterschiedlichen Aufgaben konstruktiv verschieden ausgebildet sein. Das Biogas kann entweder in Gashauben gefasst oder durch Leitbleche zum Reaktorkopf hin abgeleitet werden. Dadurch wird idealerweise erreicht, dass keine Gasblasen und kein gasbehafteter Schlamm in den Bereich der Ablaufrinnen gelangt.

Wesentliche Aufgabe aller Dreiphasentrennsysteme ist es, die Trennung der Phasen möglichst vollständig zu erreichen.

Die Schwierigkeit einer guten Dreiphasentrennung besteht insbesondere darin, die Agglomerate von den anhaftenden Gasblasen zu trennen. Spezifisch schwerere Agglomerate können durch die Anhaftung spezifisch leichterer Agglomerate gerade so austariert werden, dass das gasbehaftete Agglomerat, das im umgebenden Wasser kein Auf- oder Abtriebskräfte erfährt, quasi freischwebend vom den Strömungen im Wasser mitgetragen wird. Ein solches gasbehaftetes Agglomerat kann nun mit dem strömenden Abwasser um die gasabweisenden Leitbleche herum in den Bereich der Ablaufrinnen gelangen und mit dem Ablauf ausgetragen werden. Dieser Abtrieb kann durch die bisherigen Abscheider nicht gezielt vermieden werden. Der Abtrieb von Biomasse sollte vermieden werden, weil zum einen aktive Biomasse abtreibt und zum anderen durch die abtreibenden Feststoffe die nachfolgenden Behandlungsstufen in unnötiger Weise belastet werden.

Oftmals wurde versucht, die Strömungsgeschwindigkeit des ablaufenden Wassers weiter zu verringern, um auf diese Weise auch unter den beschriebenen ungünstigen Bedingungen einen Absetzvorgang herbeizuführen. Vielfach wurden Parallelplattensysteme eingesetzt, die eine sehr beruhigte Strömungsführung ermöglichen, aber auch hier stellte sich ein langfristiger Erfolg nicht ein.

Ein anderer Lösungsansatz, wie etwa in der EP 808 805 A1, sieht vor, dass in der Strömung Walzenströmungen gebildet werden, sogenannte Flüssigkeitswalzen. Die Strömung erhält ihre Energie aus dem aufsteigenden Biogas. Die fluidisierende Wirkung des Biogases auf das Schlammbett ist wie oben beschrieben allseits bekannt. Dabei werden die Agglomerate vorwiegend durch das Wasser fluidisiert, welches seinerseits durch die aufsteigenden Gasbläschen beschleunigt wird. Jedoch hat sich die dort vorgesehene Abscheidung der Feststoffe als nicht ausreichend herausgestellt. Das Dreiphasengemisch aus Wasser, Agglomeraten und Biogas bildet aufgrund der konstruktiven Ausbildung des Dreiphasentrennsystems in dem wasserführenden Teil des Reaktors mehr oder weniger stabile Walzenströmungen aus. Treibende Kraft einer Walzenströmung ist dabei im Wesentlichen das aufsteigende Biogas. Gemeinsames Kennzeichen dieser Walzen ist eine horizontal ausgerichtete Drehachse.

Es gibt jedoch verschiedene Belastungszustände eines Anaerobreaktors, in denen ein höherer Anteil der oben beschriebenen gasbehafteten Agglomerate gebildet werden. Das sind Zustände, in denen die Konzentration an organischen Abwasserinhaltsstoffen im Reaktor vergleichsweise gering ist. In der Folge ist die Produktion von Biogas verlangsamt oder findet in verringertem Umfang statt. Kleinere Gasbläschen sind die Folge, welche vermehrt an der Oberfläche der sie ausscheidenden Agglomerate haften bleiben und zu den oben beschriebenen Problemen führen.

Die Gasablösung kann aber nur dann mit hoher Effizienz erfolgen, wenn die mittlere Drehgeschwindigkeit der Walze eine bestimmte Mindestgeschwindigkeit erreicht. Da der Antrieb der Walze durch den Aufstieg der freigesetzten Biogasblasen erfolgt, ist eine direkte Kopplung an die aktuelle Belastungs- und Abbausituation gegeben. Prinzipiell kann dann die aktuelle Walzengeschwindigkeit drei Bereichen zugeordnet werden: 1. zu langsam bei geringer Biogasproduktion, 2. im richtigen Bereich bei einer Biogasproduktion im Auslegungsbereich, 3. zu schnell bei zu hoher Biogasproduktion. Eine zu hohe Walzengeschwindigkeit kann zu überhöhten Scherbeanspruchungen an den Agglomeraten führen. Dann wird nicht nur das anhaftende Gas abgetrennt, mitunter werden die Agglomerate selbst so in Mitleidenschaft gezogen, dass sie teilweise oder auch ganz zerbrechen. Insgesamt kann durch Scherbeanspruchungen dieser Art eine Entwicklung eingeleitet werden, die größere, sphärische Agglomerate benachteiligt und kleinvolumige, flockige Agglomerate bevorzugt.

Abwasser, insbesondere industrielles Abwasser, ist durch zeitliche Veränderungen in der Zusammensetzung gekennzeichnet. Im industriellen Bereich sind die Zusammensetzungen durch tägliche und wöchentliche Arbeitszeiten, Reinigungsintervalle und auch Kampagnenbetriebe geprägt. Auch eine mittelfristige Belastung im Auslegungsbereich ist durch stunden- oder tageweise Unter- bzw. Überlastungen geprägt.

Auf solche Belastungsschwankungen, die in der Folge durch Schwankungen in der Gasproduktion gekennzeichnet sind, kann die bisher bekannte Verfahrenstechnik nur unzureichend reagieren. Beispielsweise kann die Rezirkulation erhöht werden, um bei geringer hydraulischer Belastung die Aufströmgeschwindigkeit zu erhöhen.

Eine Aufgabe der vorliegenden Erfindung besteht nun darin, ein Verfahren und ein entsprechendes Dreiphasentrennsystem zu schaffen, wo auch bei unterschiedlichen Belastungen eine ausreichende Trennung von Biogas und Agglomeraten erfolgt und somit eine verbesserte Ablaufqualität erzielt wird.

Diese Aufgabe wird erfindungsgemäß durch ein Verfahren nach Anspruch 1 bzw. durch ein Dreiphasentrennsystem nach Anspruch 6 gelöst.

Insbesondere in Verbindung mit dem erfindungsgemäßen Abscheider kann unter Nutzung des erfindungsgemäßen Verfahrens die Drehgeschwindigkeit der Flüssigkeitswalze gezielt beeinflusst werden, sodass bei geringer Gasproduktion die Drehgeschwindigkeit erhöht sowie bei hoher Gasproduktion die Drehgeschwindigkeit erniedrigt wird. Durch die Flüssigkeitswalze wird verhindert, dass sich über dem Plattenstapel ein Gashebereffekt ausbildet.

Bei einer geringen Gasproduktion und zu geringer Drehgeschwindigkeit kann gemäß einer Ausführungsvariante der Erfindung, Gas, insbesondere Biogas, in eine seitlich des Plattenstapels angeordnete Gaseinperlung geleitet werden. Dort steigt es auf und beschleunigt die waagerecht darüber liegende Flüssigkeitswalze.

Ein an der Unterseite der unteren Platte angeordneter Strömungskeil wirkt als Strömungsabrisskante und stabilisiert die waagerecht liegende Flüssigkeitswalze dahingehend, daß alle Platten hydraulisch besser genutzt werden.

Die Drehrichtung der Flüssigkeitswalze ist durch die Kombination von Gaseinbringung und Strömungsabrisskante stabilisiert und schlägt auch bei unterschiedlichen Belastungszuständen nicht um. Selbstverständlich kann bei konstanten Verhältnissen im Schlammbettreaktor auf eine Steuerung der Drehgeschwindigkeit der Flüssigkeitswalze verzichtet werden, und daher allein mittels Plattenstapel und Strömungsabrisskante ohne Gaseinbringung der gewünschte Effekt erzielt werden.

Das eingeleitete Biogas stammt entweder aus dem Reaktorkopf und wird durch eine entsprechende Pumpe gefördert, es kann aus separaten Gebinden oder Lagerbehältern stammen, weiterhin kann z.B. Stickstoff verwendet werden, wenn noch nicht genügend Biogas zur Verfügung steht. Schließlich wird gebildetes Biogas unter der Gashaube im unteren Teil des Dreiphasentrennsystems aufgefangen. Dieses kann entweder direkt in den Reaktorkopf abgeleitet werden, oder über entsprechende Stellung der Ventile in die Gaseinperlung geleitet werden. Bei der Zuführung zur Gaseinperlung wird die Walzengeschwindigkeit in oben beschriebener Weise beschleunigt.

Wird das gebildete Biogas aber aus der Gashaube direkt in den Reaktorkopf abgeleitet, so steht insgesamt weniger Biogas zur Verfügung und der Flüssigkeitswalze wird Antriebsenergie entzogen, die Drehgeschwindigkeit verringert sich. Bei verringerter Drehgeschwindigkeit reduzieren sich die Scherbeanspruchungen und damit die Kräfte, die an den Agglomeraten wirken.

Mit dem erfindungsgemäßen Dreiphasentrennsystem wird der Rückhalt der Biomasse unter verschiedenen Belastungszuständen erreicht. Dazu wird beispielsweise an signifikanten Stellen des Abscheiders ein Teil des gebildeten Biogases gezielt abgezogen bzw. zugegeben. Das restliche Biogas steigt außerhalb des oder der Abscheider frei an die Wasseroberfläche auf und sammelt sich im Bereich des Reaktorkopfes. Weitere Ausführungen des Dreiphasentrennsystems können den abhängigen Ansprüchen entnommen werden.

Die Erfindung wird anhand der schematischen Figuren 1 bis 3 beispielhaft beschrieben.

Fig. 1 zeigt eine Seitenansicht eines Dreiphasentrennsystems, im Folgenden kurz Abscheider.

Fig. 2 zeigt den Abscheider aus Fig. 1 mit senkrechten Einbauten über den Plattenstapeln.

Fig. 3 zeigt den Abscheider aus Fig. 2 mit weiteren strömungslenkenden Einrichtungen über den Plattenstapeln.

Der Abscheider ist in einem geschlossenen Reaktor 1 angeordnet und besteht im Wesentlichen aus einer Gashaube 2, aus Plattenstapeln 3, aus Abscheiderplatten 4 und aus Rinnen 5. Über der Gashaube 2 sind zwei Plattenstapel 3 jeweils bestehend aus drei Platten 6, 7, 8 so angeordnet, dass die neben der Gashaube 2 aufsteigende Flüssigkeit auf die unterste Platte 6 trifft, entlang dieser aufsteigt und an deren Oberkante umgelenkt wird und entlang der Platten 6, 7, 8 bzw. der Gashaube 2 nach unten strömt. Die Abscheiderplatten 4 sind so angeordnet, dass deren oberes Ende über den Wasserspiegel 9 des Schlammbettreaktors 1 ragt.

An der unteren Platte 6 ist ein Strömungskeil 10 angeordnet, der als Abrisskante arbeitet und somit die bessere hydraulische Nutzung des Plattenstapels 3 bewirkt.

Die zwischen den beiden Plattenstapeln 3 angeordneten Abscheiderplatten 4 bilden einen unten offenen strömungsberuhigten keilförmigen Abscheideraum, in den das Wasser von unten eintritt. Die Rinnen 5 an der Wasseroberfläche sind so angeordnet, dass sich darin das gereinigte Wasser sammelt und abgeleitet werden kann. Um die Strömungen im Abscheideraum zu trennen, kann eine Trennwand 20 vorgesehen werden.

Das im Reaktor 1 entstehende Biogas wird zum Teil in der Gashaube 2 gesammelt, wobei sich der Wasserspiegel 21 in der Gashaube entsprechend der Gasmenge einstellt. Das Biogas wird über Leitung 12 abgezogen und kann entweder über Leitung 18, Ventil 16 und Leitung 17 durch Auslass 11 in den Reaktorkopf geleitet werden, wobei die abgezogene Gasmenge einstellbar ist. Andererseits kann über Leitung 18, Ventil 15, Leitung 19 und schließlich Leitung 13 (der Gaseinperlung) das Biogas gezielt zur Erhöhung der Drehgeschwindigkeit der Flüssigkeitswalze eingebracht werden, wobei die zugeführte Gasmenge ebenfalls einstellbar ist. Andererseits kann der Flüssigkeitswalze, falls nicht ausreichend Biogas zur Verfügung steht, über Ventil 14 und Leitung 19 Gas aus einer anderen Quelle zugeführt werden.

Im Reaktorkopf ist zumindest eine Einrichtung 24 zum Ableiten des erzeugten Biogases vorgesehen.

Der Abscheider ist, bis auf die Leitungen, aus geraden Elementen aufgebaut, die sich normal zur Zeichenebene erstrecken. Es sind normal zur Zeichenebene mehrere Leitungen 13 angeordnet, sodass deren Gasauslässe auf Geraden liegen und eine linienförmige Beaufschlagung mit Gas ermöglichen. Die Auslässe der Leitung 13 liegen nahe an der Reaktorwand 1.

Erfindungsgemäß können ein oder mehrere Abscheider an der Wasseroberfläche und parallel zu dieser angeordnet werden.

Die Erfindung nutzt die Tatsache, dass Agglomerate und anhaftende Gasbläschen in einer hochturbulenten Strömung aufgrund ihres Dichteunterschiedes und der daraus unterschiedlichen angreifenden Beschleunigungskräfte voneinander getrennt werden können.

Erfindungsgemäß werden die hochturbulenten Zone und die scharfkantige Umlenkung durch die konstruktive Anordnung eines Stapels paralleler Platten und der Abrisskante an der unteren Platte erreicht, der von der dreiphasigen Strömung durchquert werden muss, bevor sie in eine beruhigte Ablaufzone eintritt. Es ist wesentlich, dass der Plattenstapel nur zur Strömungsführung eingesetzt wird, die Strömung durch den Stapel erfolgt von oben nach unten. Ein bestimmter Anteil der austretenden Strömung gelangt in die beruhigte Ablaufzone. Ein Teil der Strömung wird um den Plattenstapel herum auf einer Kreisbahn wieder nach oben geführt und taucht zum Großteil wieder in den Plattenstapel ein. Der Plattenstapel hat keine Funktion in Bezug auf eine mögliche Sedimentation.

Der erfindungsgemäße Abscheider ist so konstruiert, dass sich neben bzw. über dem Plattenstapel herum eine Walze entwickelt und stabilisiert wird. Durch weitere Einbauten, die konstruktiv dem Plattenstapel zugeordnet sind, kann die Ausbildung der Walze unterstützt werden. Ein nicht erfindungsgemäßer Einbau konstruktiver Bauteile kann die Walzenbildung stören und unter Umständen sogar unterbinden.

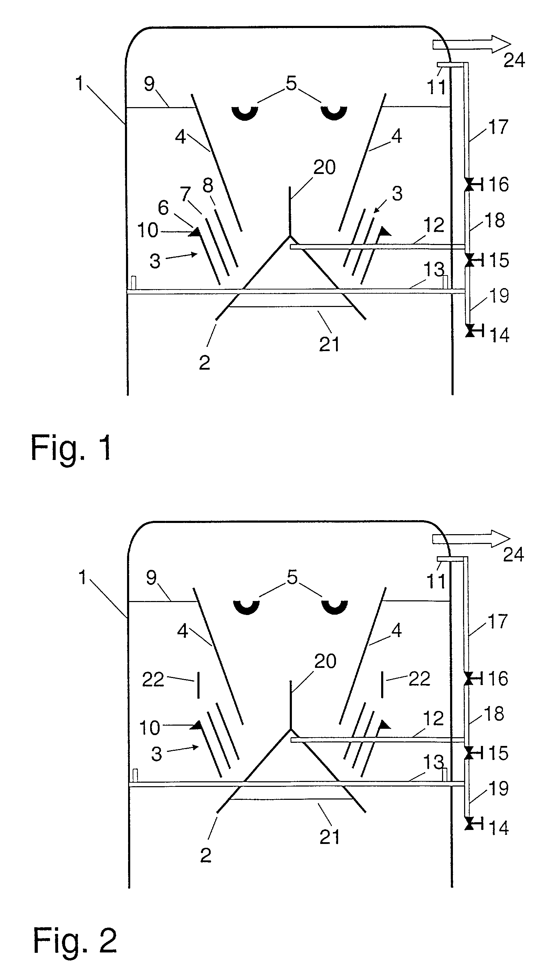

Fig. 2 zeigt ausgehend von Fig. 1 oberhalb der Plattenstapel 3, fluchtend mit der Oberkante der äußeren bzw. unteren Platte 6, jeweils eine weitere senkrecht angeordnete ebene Platte 22, die die Funktion hat, den Kern der Walze zu stabilisieren.

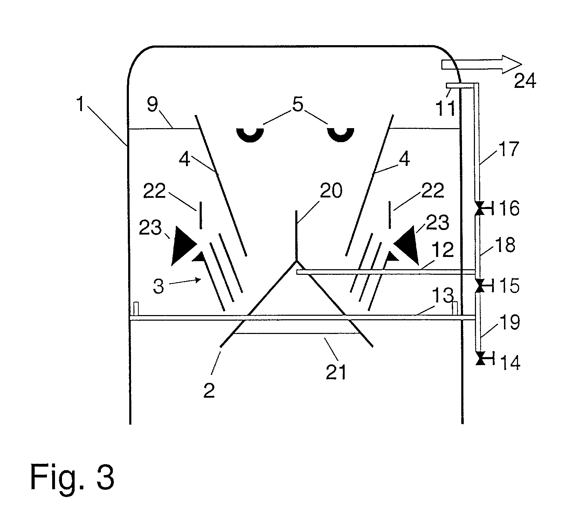

Fig. 3 zeigt ausgehend von Fig. 2 oberhalb der Plattenstapel 3 eine weitere strömungslenkende Einrichtungen 23, die dreieckig im Querschnitt ist und die Funktion hat, aufsteigende Gasblasen und abströmendes Wasser der Walze in Richtung ihres Drehsinns zuzuführen.

Auch der Einbau weiterer Leitbleche und Gashauben, wie in EP 0 808 805 A1 beschrieben, kann in Kombination mit dem erfindungsgemäßen Abscheider das erfindungsgemäße Verfahren unterstützen.

In der Kombination erfindungsgemäßer Abscheider und erfindungsgemäßes Verfahren steht eine Technik zur Verfügung, die eine gezielte Regelung des sensitiven Vorganges der Dreiphasentrennung erlaubt. Die Anströmung des Abscheiders ist nicht mehr allein von der Belastungssituation im Reaktor und dem aktuellen Abbauverhalten abhängig. Mit dem Maßnahmen der Gaszuleitung und der Gasabführung können die zur Dreiphasentrennung notwendigen Scherbeanspruchungen gezielt im optimalen Bereich eingestellt werden.

## Patentansprüche

1. Verfahren zur Trennung von Gas, Wasser und Biomasse in einem Schlammbettreaktor (1) zur anaeroben Reinigung von Abwasser durch Vergärung organischer Abwasserinhaltstoffe mittels einer fluidisierbaren, Agglomerate enthaltenden Biomasse, wobei über dem Schlammbett mittels Dreiphasentrennsystem einerseits Biomasse im System zurückgehalten, Biogas abgeleitet und behandeltes Abwasser separat aus dem Reaktor abgezogen wird und wobei zumindest eine Flüssigkeitswalze erzeugt wird, **dadurch gekennzeichnet, dass** die Drehgeschwindigkeit der Flüssigkeitswalze bei Bedarf gesteuert wird.

2. Verfahren nach Anspruch 1, **dadurch gekennzeichnet, dass** die Drehgeschwindigkeit durch Gaseinleitung (13) in Strömungsrichtung der Flüssigkeitswalze erhöht wird.

3. Verfahren nach Anspruch 1 oder 2, **dadurch gekennzeichnet, dass** die Drehgeschwindigkeit durch Einleiten von mit dem Dreiphasentrennsystem gesammeltem Biogas (13) erhöht wird.

4. Verfahren nach einem der Ansprüche 1, 2 oder 3, **dadurch gekennzeichnet, dass** die Drehgeschwindigkeit durch Abziehen von Biogas unterhalb der Flüssigkeitswalze verringert wird.

5. Verfahren nach einem der Ansprüche 1 bis 4, **dadurch gekennzeichnet, dass** die Strömung der Flüssigkeitswalze um einen Plattenstapel geführt wird und zur besseren hydraulischen Nutzung des Plattenstapels vom Plattenstapel abgelöst wird.

6. Dreiphasentrennsystem zur Trennung von Gas, Wasser und Biomasse in einem Schlammbettreaktor (1) zur anaeroben Reinigung von Abwasser durch Vergärung organischer Abwasserinhaltstoffe mittels einer fluidisierbaren, Agglomerate enthaltenden Biomasse, wobei zumindest eine Gashaube (2) zum Sammeln von Biogas, zumindest eine Einrichtung (5) zum Sammeln des ablaufenden Wassers und zumindest ein Plattenstapel (3) bestehend aus parallel in einem Abstand voneinander angeordneten und relativ zur Vertikalen geneigten geraden Platten (6, 7, 8) zur Erzeugung zumindest einer Flüssigkeitswalze vorgesehen ist, **dadurch gekennzeichnet, dass** eine Einrichtung (13) zur Steuerung der Drehgeschwindigkeit der Flüssigkeitswalze vorgesehen ist.

7. Dreiphasentrennsystem nach Anspruch 6, **dadurch gekennzeichnet, dass** im Bereich des Plattenstapels (3), insbesondere seitlich des Plattenstapels, eine Einrichtung (13) zum geregelten Einbringen von Gas so angeordnet ist, dass Gas in Strömungsrichtung der Flüssigkeitswalze eingebracht werden kann.

8. Dreiphasentrennsystem nach Anspruch 7, **dadurch gekennzeichnet, dass** in Höhe der Kante der untersten Platte (6) des Plattenstapels (3), parallel zu dieser, aber in einer Entfernung von der Kante, linienförmig Gas einbringbar ist.

9. Dreiphasentrennsystem nach Anspruch 6, 7 oder 8, **dadurch gekennzeichnet, dass** eine Leitungseinrichtung (11, 12, 16, 17, 18) vorgesehen ist, mit welcher das in der unterhalb des Plattenstapels (3) angeordneten Gashaube (2) gesammelte Biogas regelbar aus der Gashaube abgezogen werden kann.

10. Dreiphasentrennsystem nach Anspruch 9, **dadurch gekennzeichnet, dass** die Leitungseinrichtung (11, 12, 16, 17, 18) mit der Einrichtung (13) zum Einbringen von Gas verbunden ist.

11. Dreiphasentrennsystem nach einem der Ansprüche 6 bis 10, **dadurch gekennzeichnet, dass** zur besseren hydraulischen Nutzung des Plattenstapels (3) an der unteren Platte (6), insbesondere an deren oberem Ende, eine Strömungsabrisskante (10) vorgesehen ist.

12. Dreiphasentrennsystem nach einem der Ansprüche 6 bis 11, **dadurch gekennzeichnet, dass** symmetrisch bezüglich der vertikalen Symmetrieebene der Gashaube (2) oberhalb der Gashaube zwei Plattenstapel (3) angeordnet sind sowie neben der obersten Platte (8) jedes Plattenstapels eine ebene Abscheiderplatte (4) angeordnet ist, sodass über der Gashaube ein keilförmiger, nach unten offener Abscheideraum gebildet wird.

13. Dreiphasentrennsystem nach einem der Ansprüche 6 bis 12, **dadurch gekennzeichnet, dass** zur verbesserten Strömungsführung der Flüssigkeitswalze konstruktive Elemente (22, 23) seitlich neben und/oder über dem Plattenstapel (3) angeordnet sind.

14. Anordnung zur Trennung von Gas, Wasser und Biomasse in einem Schlammbettreaktor (1), **dadurch gekennzeichnet, dass** mehrere Dreiphasentrennsysteme nach einem der Ansprüche 6 bis 13 nebeneinander, insbesondere parallel zueinander, angeordnet sind.
